# EUROPÄISCHE PATENTSCHRIFT

(11) **EP 1 309 296 B1**
(45) Veröffentlichungstag und Bekanntmachung des Hinweises auf die Patenterteilung: **09.08.2006**
(21) Anmeldenummer: 01944856.2
(22) Anmeldetag: 09.07.2001
(51) Int. Cl.: A61F 2/46

(54) **VORRICHTUNG ZUR OPTIMIERUNG EINER KNIE-ENDOPROTHESE**
DEVICE FOR OPTIMIZING A KNEE ENDOPROSTHESIS
DISPOSITIF POUR OPTIMISER UNE ENDOPROTHESE DU GENOU

(30) Priorität: 19.08.2000 DE 20014377 U
(43) Veröffentlichungstag der Anmeldung: 14.05.2003
(73) Patentinhaber: Biomet Merck GmbH, 3216 Ried b. Kerzers (CH)
(72) Erfinder: CORTELLESSA, Maurizio, CH-3400 Burgdorf (CH); HERZOG, Roland, CH-4437 Waldenburg (CH); WYMENGA, Ate, B., NL-6522 Jv. Nijmegen (NL)
(74) Vertreter: Lusuardi, Werther
(86) Internationale Anmeldenummer: PCT/CH2001/000426
(87) Internationale Veröffentlichungsnummer: WO 2002/017826

(56) Entgegenhaltungen:
- EP-A- 0 720 834
- EP-A- 0 979 636
- WO-A-97/30640
- WO-A-97/30648
- CA-A- 2 201 800
- DE-U- 29 906 909
- US-A- 4 524 766
- US-A- 5 810 831

## Beschreibung

Die Erfindung betrifft eine Vorrichtung zur Bestimmung der optimalen Dimensionen einzelner Komponenten einer Knie-Endoprothese gemäss dem Oberbegriff des Patentanspruchs 1. Eine solche Vorrichtung ist aus der EP-A-O 720 834 bekannt.

Aus der US-A-4 524 766 PETERSEN ist eine Vorrichtung bekannt, welche mehrere Richt- und Führungsvorrichtungen umfasst. Eine dieser Führungsvorrichtungen umfasst eine L-förmige Platte mit einem Distanzhalter und einen Fuss, welcher am Femur ausgerichtet und befestigt wird. Diese Führungsvorrichtung dient einerseits zur Werkzeugführung bei der anterioren Femurresektion und andererseits zur Planung der Höhe der posterioren Femurresektion, wobei der Distanzhalter zur Einstellung der Distanz zwischen dem Femur und der Tibia bei gebeugtem Kniegelenk dient. Die der Dicke der zu implantierenden Tibiakomponente entsprechend einzustellende Distanz ist auf einer Skala an der Platte ablesbar. Anschliessend wird eine andere Führungsvorrichung zur distalen Resektion der Femurkondylen an der Tibia befestigt. Diese zweite Führungsvorrichtung enthält ein Führungselement, welches bezüglich der Höhe der zu implantierenden Tibiakomponente einstellbar ist.

Die Bänderspannung wird bei dieser bekannten Vorrichtung nur im gebeugten Zustand des Kniegelenkes bei der Anwendung der ersten Führungsvorrichtung berücksichtigt. Die Führungsvorrichtung für die distale Resektion der Femurkondylen bei gestrecktem Knie ist nur bezüglich der Dicke der Tibiakomponente und ohne Berücksichtigung der Bänderspannung einstellbar.

Demgegenüber liegt der Erfindung das Problem zugrunde, eine Vorrichtung zur Bestimmung der für den gesamten ROM (Range of Motion) optimalen Grössen der Komponenten einer Knie-Endoprothese zu schaffen, welche insbesondere bei Revisionen in der Kniealloarthroplastik verwendbar ist.

Als wesentliche und wählbare Grössen für den Bewegungsablauf dienen die A/P-Grösse (anterior/posteriore Abmessung) der Femurkomponente, der Dicke des Polyethylen-Zwischenteils (PE-Inlay) der Tibiakomponente, die Dicke des Distanzhalters zwischen der resezierten Femurfläche und der Femurkomponente (Spacer). Ferner lassen sich die Höhe der resultierenden Gelenkslinie und die externe Rotation des Femurimplantates ermitteln.

Die Erfindung löst die gestellte Aufgabe mit einer Vorrichtung, welche die Merkmale des Anspruchs 1 aufweist.

Die Vorrichtung wird an der distalen, resezierten Femurfläche angebracht, die als Referenz für die Vorrichtung dient. Die proximale Fläche der Tibia wird zur Bildung einer weiteren Referenzfläche auch reseziert.

Die erfindungsgemässe Vorrichtung zur Bestimmung der optimalen Dimensionen der Komponenten einer Knie-Endoprothese umfasst ein distal am Femur befestigbares Winkelelement, posterior (in Flexion) einen relativ zum Winkelelement verschiebbaren, ersten Taster und distal (in Extension) einen ebenfalls relativ zum Winkelelement verschiebbaren, zweiten Taster. Das Winkelelement umfasst eine erste Platte mit einer anterior gegen den Femur gerichteten Unterseite, einer zur Unterseite und zur Längsachse des Femur parallelen Plattenlängsachse, einem proximalen, ersten Ende und einem zweiten die Plattenlängsachse schneidenden, distal zum Femur angeordneten Ende und eine zweite, senkrecht auf der Unterseite stehende Platte mit einer senkrecht zur Plattenlängsachse ausgerichteten Zentralachse, einer an der distalen, resezierten Femurstirnfläche zur Anlage bringbaren Innenseite und einem posterior zum Femur angeordneten unteren Ende. Der erste Taster weist eine zur Unterseite im wesentlichen parallele, erste Auflagefläche zur Anlage an das proximale, resezierte Tibiaplateau im gebeugten Zustand des Kniegelenkes auf, ist parallel zur Zentralachse verschiebbar und an der zweiten Platte lösbar fixierbar. Der zweite Taster weist eine zur Innenseite im wesentlichen parallele, zweite Auflagefläche zur Anlage an das proximale, resezierte Tibiaplateau im gestreckten Zustand des Kniegelenkes auf, ist parallel zur Plattenlängsachse verschiebbar und an der ersten Platte lösbar fixierbar.

In der bevorzugten Ausführungsform der erfindungsgemässen Vorrichtung umfasst diese ein erstes Zwischenstück, welches koaxial zur Zentralachse verschiebbar zwischen dem posterioren, unteren Ende der zweiten Platte und dem ersten Taster angeordnet ist und an der zweiten Platte lösbar fixierbar ist. Ein zweites Zwischenstück ist koaxial zur Plattenlängsachse verschiebbar zwischen dem distalen, zweiten Ende der ersten Platte und dem zweiten Taster angeordnet und an der ersten Platte lösbar fixierbar. Die Zwischenstücke sind so ausgeführt, dass die Taster parallel zur Zentralrespektive Plattenlängsachse relativ zu den Zwischenstücken verschiebbar sind.

In einer weiteren Ausführungsform der erfindungsgemässen Vorrichtung umfassen die beiden Taster Positioniermittel, welche auf einer zur Zentralachse respektive Plattenängsachse parallelen Geraden angeordnet sind und axial aufeinanderfolgend, die Distanzen aⱼ aufweisen, wodurch die Auflagefläche der Taster relativ zu den Zwischenstücken in den definierten Abständen (Aᵢ = Σ aⱼ; j = 1 bis N) fixierbar sind.

Ferner können auch beide Zwischenstücke mit Positioniermitteln versehen sein, welche ebenfalls auf einer zur Plattenlängsachse respektive Zentralachse parallelen Geraden angeordnet sind und axial aufeinanderfolgend, die Distanzen bⱼ am zweiten Zwischenstück und die Distanzen cⱼ am ersten Zwischenstück aufweisen, wodurch die Zwischenstücke relativ zu den Platten in den definierten Abständen (Bᵢ = Σ bⱼ ; j = 1 bis M) respektive (Cᵢ = Σ cⱼ ; j = 1 bis Q) fixierbar sind.

Der jeweils erwünschte Betrag der interessierenden Grösse (Aᵢ, Bᵢ oder Cᵢ) ist für den Chirurgen an der Vorrichtung selbst gut lesbar. Dazu können an der Vorrichtung beispielsweise die verschiedenen Abstände bezeichnende Markierungen angebracht sein. Die Kreuzbänder werden durch die Vorrichtung nicht berücksichtigt, da sie primär für Revisionen einsetzbar sein soll.

Als Positioniermittel dienen vorzugsweise Nuten, welche so an den Tastern und Zwischenstücken angebracht sind, dass in den Platten einfügbare Schrauben mit den Nuten lösbar in Eingriff bringbar sind.

Die Einstellung der Abstände Aᵢ, Bᵢ, Cᵢ an der Vorrichtung gestattet eine gleichzeitige Bestimmung der A/P-Grösse der Femurkomponente durch die Einstellung das Abstandes Cᵢ, der Dicke des Polyethylen-Zwischenteils (PE-Inlay) der Tibiakomponente durch die Einstellung der Abstände Aᵢ, der Dicke des Distanzhalters zur Femurkomponente (Spacer) durch die Einstellung des Abstandes Bᵢ, sowie der Höhe der resultierenden Gelenkslinie und der externen Rotation des Femurimplantates durch Überprüfung der Bänderspannung des Knies in Flexions-Stellung (Femur und Tibia im 90°-Winkel zueinander) sowie Extensions-Stellung (Femur und Tibia im O°-Winkel zueinander ausgerichtet.

Die Ermittlung dieser Werte erfolgt bevor die A/P-Schnitte und die Schrägschnitte am Femur durchgeführt werden.

Eine bevorzugte Weiterbildung besteht darin, dass die Abstände Aᵢ den verschiedenen, standardisierten Dicken der Zwischenteils (PE-Inlays) der Tibia-Komponente, die Abstände Bᵢ den verschiedenen, standardisierten Dicken der Distanzhalters entsprechen und durch die Abstände Cᵢ die verschiedenen, standardisierten A/P - Grössen einer Femurkomponente der Knie-Endoprothese einstellbar sind.

Die Vorteile der erfindungsgemässen Vorrichtung liegen darin, dass die Dicke des Zwischenteils, die Dicke des Distanzhalters sowie die A/P-Grösse der Femurkomponente durch entsprechendes Einstellen der bewegbaren Teile der Vorrichtung so optimiert werden können, dass die Bänderspannung derjenigen des natürlichen Kniegelenkes entspricht.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Explosionsdarstellung einer Ausführungsform der erfindungsgemässen Vorrichtung;
Fig. 2 eine Ansicht der in Fig. 1 dargestellten erfindungsgemässen Vorrichtung;
Fig. 3 eine perspektivische Darstellung einer Knie-Endoprothese; und
Fig. 4 einen Schnitt durch die in den Fig. 1 und 2 dargestellten Ausführungsform der erfindungsgemässen Vorrichtung.

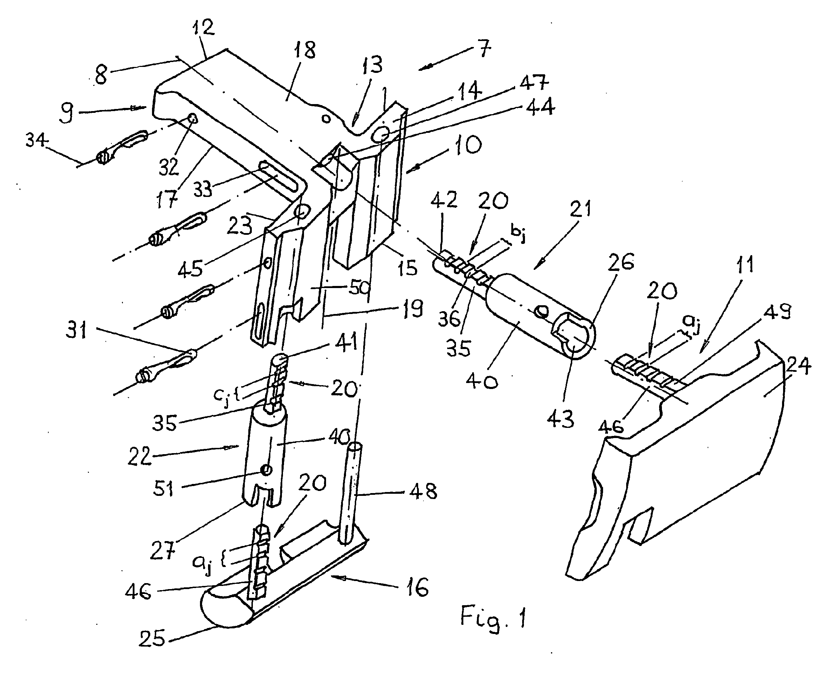

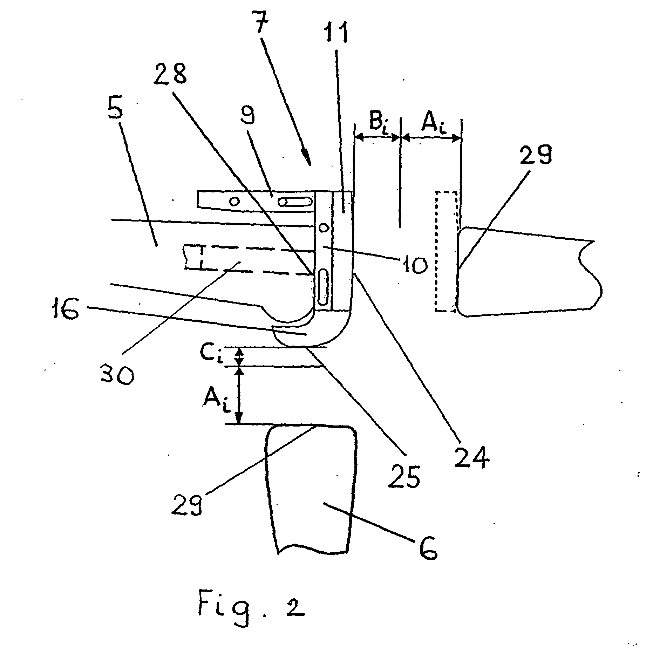

In Fig. 1 ist die bevorzugte Ausführungsform der erfindungsgemässen Vorrichtung dargestellt. Diese umfasst im wesentlichen ein Winkelelement 7, welches aus einer ersten, anterior am Femur 5 (Fig. 2) angeordneten Platte 9 mit einer Plattenlängsachse 8 und rechtwinklig dazu angeordnet einer zweiten, distal am Femur 5 (Fig. 2) angeordneten Platte 10 mit einer senkrecht auf der ersten Platte 9 stehenden Zentralachse 19 besteht, einen posterior an der zweiten Platte 10 angeordneten, parallel zur Zentralachse 19 verschiebbaren und lösbar fixierbaren ersten Taster 16 und einen distal am Winkelelement 7 angeordneten zweiten Taster 11. Die erste Platte 9 enthält posterior eine Unterseite 17, anterior eine Oberseite 18, ein erstes, proximal die Plattenlängsachse 8 schneidendes Ende 12 und ein zweites, distal die Plattenlängsachse 8 schneidendes Ende 13. Die zweite Platte 10 umfasst anterior, ein oberes Ende 14, posterior ein unteres Ende 15 und eine an das zweite Ende 13 angrenzende, im wesentlichen ebene Innenseite 23, so dass das Winkelelement 7 so mit dem Femur 5 verbindbar ist, dass die Innenseite 23 an der distalen Femurstirnfläche 28 zur Anlage bringbar ist und die erste Platte 9 anterior zum Femur 5 mit parallel zur Längsachse des Femur 5 verlaufender Plattenlängsachse 8 angeordnet ist. Am Femur 5 befestigt wird das Winkelelement 7 mittels eines intramedullären Stabes 30 (Fig. 2), welcher frei schwenkbar in den intramedullären Kanal des Femur 5 einbringbar ist. Der erste Taster 16 enthält eine zur Unterseite 17 der ersten Platte 9 im wesentlichen parallele, erste Auflagefläche 25, welche im gebeugten Zustand des Kniegelenkes am Tibiaplateau 29 zur Anlage bringbar ist. Der zweite Taster 11 ist am zweiten Ende 13 der ersten Platte 9 parallel zur Plattenlängsachse 8 verschiebbar und lösbar fixierbar angeordnet und enthält eine zur Innenseite 23 der zweiten Platte 10 im wesentlichen parallele, zweite Auflagefläche 24, welche im gestreckten Zustand des Kniegelenkes am Tibiaplateau 29 zur Anlage bringbar ist.

Ferner umfasst die Vorrichtung ein erstes Zwischenstück 22, welches eine gegen den ersten Taster 16 gerichtete untere Stirnfläche 27 aufweist, koaxial zur Zentralachse 19 verschiebbar zwischen dem unteren Ende 15 der zweiten Platte 10 und dem ersten Taster 16 angeordnet ist und an der zweiten Platte 10 lösbar fixierbar ist, so dass das Zwischenstück 22 posterior relativ zur zweiten Platte 10 und der erste Taster 16 relativ zum ersten Zwischenstück 22 und somit auch zur zweiten Platte 10 verschiebbar ist.

Analog ist zwischen dem zweiten Ende 13 der ersten Platte 9 und dem zweiten Taster 11 ein zweites Zwischenstück 21 eingefügt, welches eine gegen den zweiten Taster 11 gerichtete vordere Stirnfläche 26 aufweist, koaxial zur Plattenlängsachse 8 verschiebbar angeordnet und an der ersten Platte 9 lösbar fixierbar ist, so dass das zweite Zwischenstück 21 distal relativ zur ersten Platte 9 und der zweite Taster 11 relativ zum ersten Zwischenstück 21 verschiebbar ist.

Der unteren Stirnfläche 27 entgegengesetzt hat das erste Zwischenstück 22 ein oberes Ende 41 und umfasst axial aneinandergrenzend zwischen der unteren Stirnfläche 27 und dem oberen Ende 41 eine Hülse 40 und einen Zapfen 35. Die Hülse 40 grenzt an die untere Stirnfläche 27 und hat eine Hülsenbohrung 43, welche gegen die untere Stirnfläche 27 offen ist. Analog dazu hat das zweite Zwischenstück 21 der vorderen Stirnfläche 26 entgegengesetzt ein hinteres Ende 42 und umfasst axial aneinandergrenzend zwischen der vorderen Stirnfläche 26 und dem hinteren Ende 42 eine Hülse 40 und einen Zapfen 35, wobei die Hülse 40 an die vordere Stirnfläche 26 grenzt und die Hülsenbohrung 43 gegen die vordere Stirnfläche 26 offen ist. Zur axial verschiebbaren Aufnahme des zweiten Zwischenstückes 21 enthält die erste Platte 9 koaxial zur Plattenlängsachse 8 eine erste Führungsbohrung 44, während die zweite Platte 10 parallel zur Zentralachse 19 eine zweite Führungsbohrung 45 zur axial verschiebbaren Aufnahme des ersten Zwischenstückes 22 und ebenfalls parallel zur Zentralachse 19 eine weitere Bohrung 47 zur axial verschiebbaren Aufnahme eines mit dem ersten Taster 11 verbundenen Stiftes 48 umfasst. Am ersten und am zweiten Taster 16;11 sind der ersten, respektive der zweiten Auflagefläche 25;24 gegenüberliegend je einen Führungsbolzen 46 angebracht, welche in den Hülsenbohrungen 43 axial verschiebbar aufnehmbar sind, so dass die erste Platte 9, das zweite Zwischenstück 21 und der zweite Taster 11 bezüglich der Plattenlängsachse 8 axial teleskopierbar sind. Die zweite Platte 10, das erste Zwischenstück 22 und der erste Taster 16 dagegen sind bezüglich der Zentralachse 19 axial teleskopierbar.

Der zweite Taster 11 umfasst im weiteren Positioniermittel 20, welche auf einer zur Plattenlängsachse 8 parallelen Geraden angeordnet sind und axial aufeinanderfolgend, die Distanzen aⱼ aufweisen. Mittels dieser Positioniermittel 20 lässt sich die zweite Auflagefläche 24 des zweiten Tasters 11 relativ zur vorderen Stirnfläche 26 des zweiten Zwischenstückes in den definierten Abständen (Aᵢ = Σ aⱼ; j = 1 bis N) fixieren (Fig. 2).

Analog dazu umfasst der erste Taster 16 Positioniermittel 20, welche auf einer zur Zentralachse 19 parallelen Geraden angeordnet sind und axial aufeinanderfolgend, die Distanzen aⱼ aufweisen, wodurch die erste Auflagefläche 25 des ersten Tasters 16 relativ zur unteren Stirnfläche 27 des ersten Zwischenstückes 22 in den definierten Abständen (Aᵢ = Σ aⱼ; j = 1 bis N) fixierbar ist (Fig. 2).

Die Zapfen 35 an den ersten und zweiten Zwischenstücken 22;21 sowie die Führungsbolzen 46 an den ersten und zweiten Tastern 16;11 weisen einen kreissegmentförmigen Querschnitt auf, wobei die ebenen Flächen 49 gegen die Oberseite 18 der ersten Platte 9 respektive gegen die Aussenseite 50 der zweiten Platte 10 gerichtet sind. In diese Flächen 49 vertieft eingelassen sind Nuten 36, welche die Positioniermittel 20 bilden und quer zur Plattenlängsachse 8 respektive zur Zentralachse 19 verlaufen. Die axiale Arretierung der ersten und zweiten Zwischenstücke 22;21 relativ zu den ersten respektive zweiten Platten 9;10 erfolgt durch Arretierstifte 31, welche in ersten Querbohrungen 32 mit quer zur Plattenlängsachse 8 respektive Zentralachse 19 verlaufenden Bohrungsachsen 34 in den ersten und zweiten Platten 9; 10 axial verschiebbar gelagert und mit den Nuten 36 an den Zapfen 35 in Eingriff bringbar sind. Ebenso erfolgt die axiale Arretierung der ersten und zweiten Taster 16;11 relativ zu den ersten und zweiten Zwischenstücken 22;21 durch solche Arretierstifte 31, welche in zweiten Querbohrungen 51 in den ersten und zweiten Zwischenstücken 22;21 axial verschiebbar gelagert und mit den Nuten 36 an den Führungsbolzen 46 in Eingriff bringbar sind. Damit die ersten und zweiten Zwischenstücke 22;21 mit arretierten ersten und zweiten Tastern 16;11 dennoch relativ zu den ersten und zweiten Platten 9;10 verschiebbar sind, sind in den ersten und zweiten Platten 9;10 Langlöcher 33 mit zur Plattenlängsachse 8 respektive zur Zentralachse 19 parallelen Längsseiten zur Aufnahme der Arretierstifte 31 angebracht.

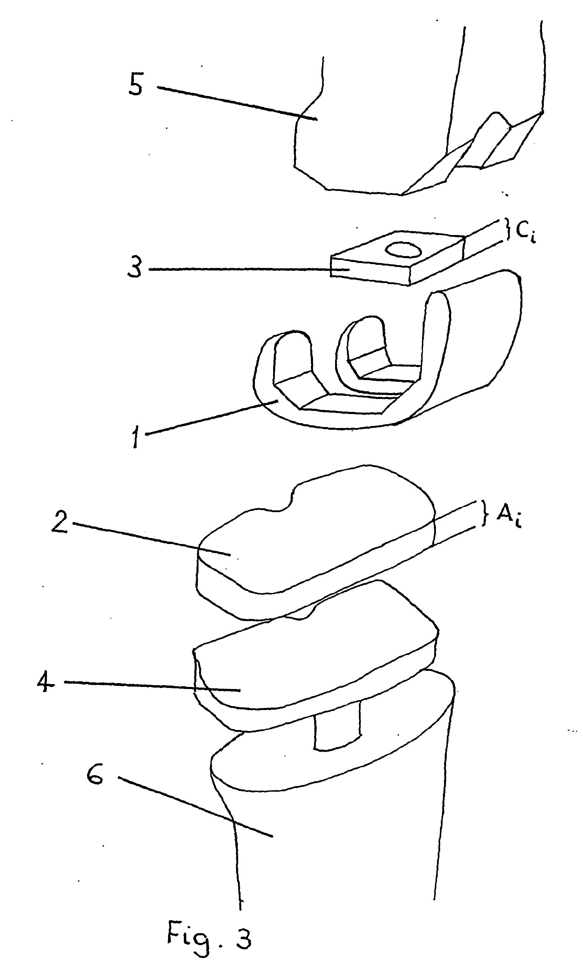

Die Abstände Aᵢ entsprechen den verschiedenen, standardisierten Dicken eines Zwischenteils 2 der Tibia-Komponente einer Knie-Endoprothese (Fig. 3).

Das zweite Zwischenstück 21 umfasst ebenfalls Positioniermittel 20, welche auf einer zur Plattenlängsachse 8 parallelen Geraden angeordnet sind und axial aufeinanderfolgend, die Distanzen bⱼ aufweisen. Damit ist die vordere Stirnfläche 26 des zweiten Zwischenstückes 21 relativ zum zweiten Ende 13 der ersten Platte 9 in den definierten Abständen (Bᵢ = Σ bⱼ ; j = 1 bis M) einstellbar. Diese Abstände Bᵢ entsprechen den verschiedenen, standardisierten Dicken eines Distanzhalters 3 zur Femurkomponente 1 einer Knie-Endoprothese.

Analog dazu umfasst das erste Zwischenstück 22 Positioniermittel 20, welche auf einer zur Zentralachse 19 parallelen Geraden angeordnet sind und axial aufeinanderfolgend, die Distanzen cⱼ aufweisen. Dadurch ist die untere Stirnfläche 27 des ersten Zwischenstückes 22 relativ zum unteren Ende 15 der zweiten Platte 10 in den definierten Abständen (Cᵢ = Σ cⱼ ; j = 1 bis Q) fixierbar, wobei die Abstände Cᵢ den verschiedenen, standardisierten A/P - Grössen 30 einer Femurkomponente 1 einer Knie-Endoprothese entsprechen.

In Fig. 3 ist eine Ausführungsform einer Knie-Endoprothese zusammen mit einem Femur 5 und einer Tibia 6 dargestellt. Die Knie-Endoprothese setzt sich aus einer Femurkomponente 1 mit einer wählbaren A/P-Grösse 30 (Anterior/Posterior-Grösse), einem distal zwischen Femur 5 und Femurkomponente 1 anbringbaren Distanzhalter 3, einer Tibiakomponente 4 und einem Zwischenstück 2 zwischen Tibiakomponente 4 und Femurkomponente 1 zusammen. Die bezüglich der Knochenlängsachse axial gemessene Dicke Aᵢ des Zwischenstückes 2, die ebenfalls bezüglich der Knochenlängsachse axial gemessene Distanz Cᵢ des Distanzhalters 3 und die anterior/posterior senkrecht zur Knochenlängsachse gemessene Anterior/Posterior-Grösse 30 der Femurkomponente 1 stellen diejenigen Abmessungen der Knie-Endoprothese dar, welche mittels der erfindungsgemässen Vorrichtung ermittelbar sind.

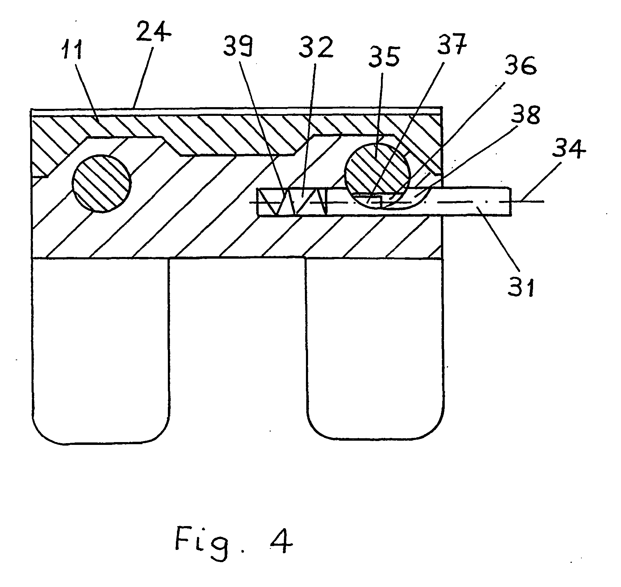

Fig. 4 zeigt beispielhaft einen der Arretierstifte 31, welcher in der ersten Querbohrung 32 in der zweiten Platte 10 koaxial zur Bohrungsachse 34 verschiebbar gelagert ist. Am Grund dieser ersten Querbohrung 32 ist eine Feder 39 eingefügt, welche den Arretierstift 31 gegen den Zapfen 35 presst. Am Arretierstift 31 ist eine radiale Vertiefungen 38 mit einem gegenüber dieser Vertiefung 38 vorstehenden Nocken 37 angebracht. Der Nocken 37 erstreckt sich nur über einen Teil der axialen Länge der Vertiefung 38. In einer ersten Position wird der Arretierstift 31 durch die Federkraft der Feder 39 gegen den Zapfen 35 gedrückt, so dass die den Zapfen 35 peripher teilweise umschliessende Vertiefung 38 relativ zum Zapfen 35 so weit axial verschoben wird, dass der Nocken 37 mit einer der Nuten 36 im Eingriff steht. In einer zweiten Position, in welche der Arretierstift 31 beispielsweise von Hand gegen die Federkraft der Feder 39 axial verschoben wird, ist die den Zapfen 35 teilweise umschliessende Vertiefung 38 relativ zum Zapfen 35 so weit verschoben, dass der Nocken 37 aus der Nute 36 bezüglich der Bohrungsachse 34 axial ausrastet und der Zapfen 35 parallel zur Zentralachse 19 verschiebbar ist. Die an diesem Beispiel erläuterte Ausführung des Arretierstiftes 31 mit Vertiefung 38, Nocken 37 und Feder 39 gilt auch für die anderen an der erfindungsgemässen Vorrichtung eingesetzten Arretierstifte 31 (Fig. 1).

## Patentansprüche

1. Vorrichtung zur Bestimmung der optimalen Dimensionen der Komponenten einer Knie-Endoprothese, mit
A) einem Winkelelement (7), welches eine erste Platte (9) mit einer Plattenlängsachse (8) und rechtwinklig dazu angeordnet eine zweite Platte (10) mit einer senkrecht auf der ersten Platte (9) stehenden Zentralachse (19) umfasst; und
B) einem parallel zur Zentralachse (19) verschiebbar und lösbar fixierbar an der zweiten Platte (10) angeordneten, ersten Taster (16) mit einer zur ersten Platte (9) im wesentlichen parallelen, ersten Auflagefläche (25) zur Anlage an ein Tibiaplateau (29) im gebeugten Zustand eines Kniegelenkes, wobei
C) die erste Platte (9) eine Unterseite (17), eine Oberseite (18), ein erstes, die Plattenlängsachse (8) schneidendes Ende (12) und ein zweites, die Plattenlängsachse (8) schneidendes Ende (13) umfasst;
D) die zweite Platte (10) ein oberes Ende (14), ein unteres Ende (15) und eine an das zweite Ende (13) angrenzende, im wesentlichen ebene Innenseite (23) umfasst, so dass das Winkelelement (7) so mit einem Femur (5) verbindbar ist, dass die Innenseite (23) an der distalen Femurstimfläche (28) zur Anlage bringbar ist und die erste Platte (9) anterior am Femur (5) mit parallel zur Längsachse des Femur (5) verlaufender Plattenlängsachse (8) angeordnet ist; und
E) die Vorrichtung einen zweiten Taster (11) umfasst, welcher eine zur Innenseite (23) im wesentlichen parallele, zweite Auflagefläche (24) zur Anlage an ein Tibiaplateau (29) im gestreckten Zustand des Kniegelenkes aufweist, am zweiten Ende (13) parallel zur Plattenlängsachse (8) verschiebbar und an der ersten Platte (9) lösbar fixierbar ist,
**dadurch gekennzeichnet, dass** die Vorrichtung
F) ein erstes Zwischenstück (22) umfasst, welches eine gegen den ersten Taster (16) gerichtete untere Stirnfläche (27) aufweist, koaxial zur Zentralachse (19) verschiebbar zwischen dem unteren Ende (15) und dem ersten Taster (16) angeordnet ist und an der zweiten Platte (10) lösbar fixierbar ist, und dass der erste Taster (16) relativ zum ersten Zwischenstück (22) verschiebbar ist; und
G) ein zweites Zwischenstück (21) umfasst, welches eine gegen den zweiten Taster (11) gerichtete vordere Stirnfläche (26) aufweist, koaxial zur Plattenlängsachse (8) verschiebbar zwischen dem zweiten Ende (13) und dem zweiten Taster (11) angeordnet ist und an der ersten Platte (9) lösbar fixierbar ist, und dass der zweite Taster (11) relativ zum zweiten Zwischenstück (21) verschiebbar ist.

2. Vorrichtung nach Anspruch 1, **dadurch gekennzeichnet, dass** der zweite Taster (11) Positioniermittel (20) umfasst, welche auf einer zur Plattenlängsachse (8) parallelen Geraden angeordnet sind und axial aufeinanderfolgend, die Distanzen aⱼ aufweisen, wodurch die zweite Auflagefläche (24) relativ zur vorderen Stirnfläche (26) in den definierten Abständen (Aᵢ = Σ aⱼ ; j = 1 bis N) fixierbar ist.

3. Vorrichtung nach Anspruch 1 oder 2, **dadurch gekennzeichnet, dass** der erste Taster (16) Positioniermittel (20) umfasst, welche auf einer zur Zentralachse. (19) parallelen Geraden angeordnet sind und axial aufeinanderfolgend, die Distanzen aⱼ aufweisen, wodurch die erste Auflagefläche (25) relativ zur unteren Stirnfläche (27) in den definierten Abständen (Aᵢ = E aⱼ; j = 1 bis N) fixierbar ist.

4. Vorrichtung nach Anspruch 1, **dadurch gekennzeichnet, dass** das zweite Zwischenstück (21) Positioniermittel (20) umfasst, welche auf einer zur Plattenlängsachse (8) parallelen Geraden angeordnet sind und axial aufeinanderfolgend, die Distanzen bⱼ aufweisen, wodurch die vordere Stirnfläche (26) relativ zum zweiten Ende (13) in den definierten Abständen (Bᵢ = E bⱼ ; j = 1 bis M) fixierbar ist.

5. Vorrichtung nach Anspruch 1, **dadurch gekennzeichnet, dass** das erste Zwischenstück (22) Positioniermittel (20) umfasst, welche auf einer zur Zentralachse (19) parallelen Geraden angeordnet sind und axial aufeinanderfolgend, die Distanzen cⱼ aufweisen, wodurch die untere Stirnfläche (27) relativ zum unteren Ende (15) in den definierten Abständen (Cᵢ = Σ Cⱼ ; j = 1 bis Q) fixierbar ist.

6. Vorrichtung nach einem der Ansprüche 1 bis 5, **dadurch gekennzeichnet, dass** die Abstände Aᵢ den verschiedenen, standardisierten Dicken eines Zwischenteils (2) der Tibia-Komponente einer Knie-Endoprothese entsprechen.

7. Vorrichtung nach einem der Ansprüche 4 bis 6, **dadurch gekennzeichnet, dass** die Abstände Bᵢ den verschiedenen, standardisierten Dicken eines Distanzhalters (3) zur Femurkomponente (1) einer Knie-Endoprothese entsprechen.

8. Vorrichtung nach einem der Ansprüche 5 bis 7, **dadurch gekennzeichnet, dass** die Abstände Cᵢ den verschiedenen, standardisierten A/P - Grössen (30) einer Femurkomponente (1) einer Knie-Endoprothese entsprechend einstellbar sind.

9. Vorrichtung nach einem der Ansprüche 1 bis 8, **dadurch gekennzeichnet, dass**
A) das erste Zwischenstück (22) ein oberes Ende (41) sowie axial aneinandergrenzend zwischen der unteren Stirnfläche (27) und dem oberen Ende (41) eine Hülse (40) und einen Zapfen (35) umfasst, wobei die Hülse (40) an die untere Stirnfläche (27) grenzt und die Hülsenbohrung (43) gegen die untere Stimfläche (27) offen ist;
B) das zweite Zwischenstück (21) ein hinteres Ende (42) sowie axial aneinandergrenzend zwischen der vorderen Stimfläche (26) und dem hinteren Ende (42) eine Hülse (40) und einen Zapfen (35) umfasst, wobei die Hülse (40) an die vordere Stirnfläche (26) grenzt und die Hülsenbohrung (43) gegen die vordere Stirnfläche (26) offen ist;
C) die erste Platte (9) koaxial zur Plattenlängsachse (8) eine erste Führungsbohrung (44) zur axial verschiebbaren Aufnahme des zweiten Zwischenstückes (21) umfasst;
D) die zweite Platte (10) parallel zur Zentralachse (19) eine zweite Führungsbohrung (45) zur axial verschiebbaren Aufnahme des ersten Zwischenstückes (22) umfasst; und
E) der erste und der zweite Taster (16;11) der ersten respektive zweiten Auflagefläche (25;24) gegenüberliegend je einen Führungsbolzen (46) umfassen, wobei
F) die Führungsbolzen (46) in den Hülsenbohrungen (43) axial verschiebbar aufnehmbar sind.

10. Vorrichtung nach Anspruch 9, **dadurch gekennzeichnet, dass**
A) an den Zapfen (35) sowie an den Führungsbolzen (46) Nuten (36) eingelassen sind, welche die Positioniermittel (20) bilden und quer zur Plattenlängsachse (8) respektive zur Zentralachse (19) verlaufen;
B) die axiale Arretierung der ersten und zweiten Zwischenstücke (22;21) relativ zu den ersten respektive zweiten Platten (9;10) durch Arretierstifte (31) erfolgt, welche in ersten Querbohrungen (32) mit quer zur Plattenlängsachse (8) respektive Zentralachse (19) verlaufenden Bohrungsachsen (34) in den ersten und zweiten Platten (9;10) axial verschiebbar gelagert und mit den Nuten (36) an den Zapfen (35) in Eingriff bringbar sind;
C) die axiale Arretierung der ersten und zweiten Taster (16;11) relativ zu den ersten und zweiten Zwischenstücken (22;21) durch Arretierstifte (31) erfolgt, welche in zweiten Querbohrungen (51) in den ersten und zweiten Zwischenstücken (22;21) axial verschiebbar gelagert und mit den Nuten (36) an den Führungsbolzen (46) in Eingriff bringbar sind;
D) in den ersten und zweiten Platten (9;10) Langlöcher (33) mit zur Plattenlängsachse (8) respektive zur Zentralachse (19) parallelen Längsseiten zur Aufnahme der Arretierstifte (31) angebracht sind, so dass die ersten und zweiten Zwischenstücke (22;21) mit arretierten ersten und zweiten Tastern (16;11) dennoch relativ zu den ersten und zweiten Platten (9; 1 0) verschiebbar sind; und
E) die Arretierstifte (31) radiale Vertiefungen (38) mit gegenüber diesen Vertiefungen (38) vorstehenden Nocken (37) umfassen, wobei die Nocken (37) nur einen Teil der axialen Länge der Vertiefungen (38) einnehmen, so dass die Arretierstifte (31) in eine erste Position, in welcher die Nocken (37) mit den Nuten (36) im Eingriff stehen, und in eine zweite Position, in welcher die Nocken (37) durch axiales Verschieben der Arretierstifte (31) aus den Nuten (36) axial ausrasten und die Zapfen (35) respektive Führungsbolzen (46) koaxial zur Plattenlängsachse (8) respektive parallel zur Zentralachse (19) verschiebbar sind.

## Claims

1. A device for determining the optimal dimensions of the components of a knee endoprosthesis, including
A) an angular element (7) which comprises a first plate (9) having a plate longitudinal axis (8) and, arranged at a right angle thereto, a second plate (10) having a central axis (19) extending perpendicularly to the first plate (9); and
B) a first calliper (16) which is releasably fastened on the second plate (10) in a manner that permits it to be displaced parallel to the central axis (19), and which has a bearing surface (25) extending essentially parallel to the first plate (9) and designed to rest against a tibial plateau (29) when a knee joint is bent,
C) said first plate (9) comprising a bottom surface (17), a top surface (18), a first end portion (12) intersecting the plate longitudinal axis (8), and a second end portion (13) intersecting the plate longitudinal axis (8);
D) the second plate (10) comprising a top end portion (14), a bottom end portion (15), and a substantially planar, inner side (23) adjoining the second end portion (13), so that the angular element (7) may be connected to the femur (5) in such a way that the inner side (23) may be brought to rest against the distal end face (28) of the femur and the first plate (9) is arranged anteriorly to the femur (5) with the plate longitudinal axis (8) extending parallel to the longitudinal axis of the femur (5); and
E) comprises a second calliper (11) which has a second bearing surface (24) extending substantially parallel to the inner side (23) and designed to rest against a tibial plateau (29) when the knee joint is straightened, and which on the second end portion (13) is displaceable parallel to the longitudinal axis (8) and releasably fastenable on the first plate (9),
**characterised in that** the device
comprises a first inlay (22) which has a bottom end face (27) facing the first calliper (16), and is arranged between the bottom end portion (15) and the first calliper (16) in such a way as to be displaceable coaxially to the central axis (19) and is releasably fastenable on the second plate (10), and **in that** the first calliper (16) is displaceable relative to the first inlay (22); and
a second inlay (21) which has an anterior end face (26) facing the second calliper (11), and is arranged between the second end portion (13) and the second calliper (11) in such a way as to be displaceable coaxially to the plate longitudinal axis (8) and is releasably fastenable on the first plate (9), and **in that** the second calliper (11) is displaceable relative to the second inlay (21).

2. The device of claim 1, **characterised in that** the second calliper (11) comprises positioning means (20) which are arranged on a straight line extending parallel to the plate longitudinal axis (8) and are spaced apart from one another in axial succession by the distances aⱼ, whereby the second bearing surface (24) is fastenable at the defined distances (Aᵢ = Σ aⱼ; j = 1 to N) relative to the anterior end face (26).

3. The device of claim 1 or 2, **characterised in that** the first calliper (16) comprises positioning means (20) which are arranged on a straight line extending parallel to the central axis (19) and are spaced apart from one another in axial succession by the distances aⱼ, whereby the first bearing surface (25) is fastenable at the defined distances (Aᵢ = Σ aⱼ; j = 1 to N) relative to the bottom end face (27).

4. The device of claim 1, **characterised in that** the second inlay (21) comprises positioning means (20) which are arranged on a straight line extending parallel to the plate longitudinal axis (8) and are spaced apart from one another in axial succession by the distances bⱼ, whereby the anterior end face (26) is fastenable at the defined distances (Bᵢ = Σ bⱼ; j = 1 to M) relative to the second end portion (13).

5. The device of claim 1, **characterised in that** the first inlay (22) comprises positioning means (20) which are arranged on a straight line extending parallel to the central axis (19) and are spaced apart from one another in axial succession by the distances cⱼ, whereby the bottom end face (27) is fastenable at the defined distances (Cᵢ = Σ cⱼ; j = 1 to Q) relative to the bottom end portion (15).

6. The device as claimed in any of the claims 1 to 5, **characterised in that** the distances Aᵢ correspond to the different, standardised thicknesses of an inlay (2) of the tibial component of a knee endoprosthesis.

7. The device as claimed in any of the claims 4 to 6, **characterised in that** the distances Bᵢ correspond to the different, standardised thicknesses of a spacer (3) for the femoral component (1) of a knee endoprosthesis.

8. The device as claimed in any of the claims 5 to 7, **characterised in that** the distances Cᵢ are adjustable corresponding to the different, standardised A/P sizes (30) of a femoral component (1) of a knee endoprosthesis.

9. A device as claimed in any of the claims 1 to 8, **characterised in that**
A) the first inlay (22) comprises a top end portion (41) and, extending between the bottom end face (27) and the top end portion (41) and arranged axially adjacent to one another, a sleeve (40) and a pin (35), the sleeve (40) adjoining the bottom end face (27) and the sleeve bore (43) being open towards the bottom end face (27);
B) the second inlay (21) comprises a posterior end portion (42) and, extending between the anterior end face (26) and the posterior end portion (42) and arranged axially adjacent to one another, a sleeve (40) and a pin (35), the sleeve (40) adjoining the anterior end face (26) and the sleeve bore (43) being open towards the anterior end face (26);
C) the first plate (9) comprises a first guide bore (44) extending coaxially to the plate longitudinal axis (8) and designed to receive the second inlay (21) in such a way as to be axially displaceable;
D) the second plate (10) comprises a second guide bore (45) extending parallel to the central axis (19) and designed to receive the first inlay (22) in such a way as to be axially displaceable; and
E) the first and second callipers (16;11) comprise each a guide bolt (46) facing the first and second bearing surfaces (25;24), respectively,
F) the guide bolts (46) being receivable, by way of axial displacement, within the sleeve bores (43).

10. A device as claimed in claim 9, **characterised in that**
A) the pins (35) and the guide bolts (46) are provided with grooves (36) which form the positioning means (20) and extend crosswise to the plate longitudinal axis (8) and the central axis (19), respectively;
B) the axial arresting of the first and second inlays (22;21) relative to the first and second plates (9;10), respectively, is realised by means of location pins (31) which are lodged in an axially displaceable manner within the first and second plates (9;10) by means of first transverse bores (32) having bore axes (34) extending crosswise to the plate longitudinal axis (8) and to the central axis (19), respectively, said location pins being capable of engaging with the grooves (36) formed in the pins (35);
C) the axial arresting of the first and second callipers (16:11) relative to the first and second inlays (22:21) is realised by means of location pins (31) which are lodged in an axially displaceable manner within second transverse bores (51) formed in the first and second inlays (22;21) and may be brought to engage with the grooves (36) formed in the guide bolts (46);
D) the first and second plates (9;10) are provided with elongate holes (33) having their longitudinal dimensions parallel to the plate longitudinal axis (8) and to the central axis (19), respectively, and which serve for receiving the location pins (31), so that the first and second inlays (22;21) with arrested first and second callipers (16;11) are nonetheless displaceable relative to the first and second plates (9; 10); and
E) the location pins (31) comprise radial recesses (38) including cams (37) protruding from these recesses (38), the cams (37) extending only over part of the axial length of the recesses (38), so that the location pins (31) may be moved into a first position, in which the cams (37) are in engagement with the grooves (36), and into a second position, in which the cams (37), by the axial displacement of the location pins (31), are axially released from the grooves (36), and the pins (35) and guide bolts (46) are displaceable coaxially to the plate longitudinal axis (8) and parallel to the central axis (19), respectively.

## Revendications

1. Dispositif pour déterminer les dimensions optimales des composants d'une endoprothèse du genou, comprenant
A) un élément angulaire (7), lequel comprend une première plaque (9) ayant un axe longitudinal de plaque (8) et, perpendiculairement à celle-ci, une deuxième plaque (10) ayant un axe central (19) situé perpendiculairement à la première plaque (9) ; et
B) un premier palpeur (16), pouvant être déplacé parallèlement à l'axe central (19) et pouvant être monté de manière amovible sur la deuxième plaque (10), ayant une première surface d'appui (25) essentiellement parallèle à la première plaque (9) pour l'appui contre un plateau tibial (29) lorsqu'une articulation du genou est en flexion, dans lequel
C) la première plaque (9) comprend une face inférieure (17), une face supérieure (18), une première extrémité (12) coupant l'axe longitudinal de plaque (8) et une deuxième extrémité (13) coupant l'axe longitudinal de plaque (8) ;
D) la deuxième plaque (10) comprend une extrémité supérieure (14), une extrémité inférieure (15) et une face interne (23) essentiellement plane contiguë à la deuxième extrémité (13), de sorte que l'élément angulaire (7) peut être lié à un fémur (5) de telle manière que la face interne (23) peut être appuyée sur la face frontale distale du fémur (28) et la première plaque (9) est disposée antérieurement au fémur (5) avec l'axe longitudinal de plaque (8) s'étendant parallèlement à l'axe longitudinal du fémur (5) ; et
E) le dispositif comprend un deuxième palpeur (11), lequel présente une deuxième surface d'appui (24), essentiellement parallèle à la face interne (23), pour l'appui sur un plateau tibial (29) lorsque l'articulation du genou est en extension, peut être déplacé au niveau de la deuxième extrémité (13) parallèlement à l'axe longitudinal de plaque (8) et peut être monté de manière amovible sur la première plaque (9),
**caractérisé en ce que** le dispositif
F) comprend une première pièce intermédiaire (22), laquelle présente une face frontale inférieure (27) tournée vers le premier palpeur (16), est disposée de manière à pouvoir être déplacée coaxialement à l'axe central (19) entre l'extrémité inférieure (15) et le premier palpeur (16) et peut être montée de manière amovible sur la deuxième plaque (10), et **en ce que** le premier palpeur (16) peut être déplacé par rapport à la première pièce intermédiaire (22) ; et
G) comprend une deuxième pièce intermédiaire (21), laquelle présente une face frontale avant (26) tournée vers le deuxième palpeur (11), est disposée de manière à pouvoir être déplacée coaxialement à l'axe longitudinal de plaque (8) entre la deuxième extrémité (13) et le deuxième palpeur (11) et peut être montée de manière amovible sur la première plaque (9), et **en ce que** le deuxième palpeur (11) peut être déplacé par rapport à la deuxième pièce intermédiaire (21).

2. Dispositif selon la revendication 1, **caractérisé en ce que** le deuxième palpeur (11) comprend des moyens de positionnement (20), lesquels sont disposés sur une droite parallèle à l'axe longitudinal de plaque (8) et présentent successivement sur l'axe les distances aⱼ, de sorte que la deuxième surface d'appui (24) peut être bloquée par rapport à la face frontale avant (26) à des intervalles définis (Aᵢ = Σ aj ; j = 1 à N).

3. Dispositif selon la revendication 1 ou 2, **caractérisé en ce que** le premier palpeur (16) comprend des moyens de positionnement (20), lesquels sont disposés sur une droite parallèle à l'axe central (19) et présentent successivement sur l'axe les distances aⱼ, de sorte que la première surface d'appui (25) peut être bloquée par rapport à la face frontale inférieure (27) à des intervalles définis (Aᵢ = Σ aⱼ ; j = 1 à N).

4. Dispositif selon la revendication 1, **caractérisé en ce que** la deuxième pièce intermédiaire (21) comprend des moyens de positionnement (20), lesquels sont disposés sur une droite parallèle à l'axe longitudinal de plaque (8) et présentent successivement sur l'axe les distances bj, de sorte que la face frontale avant (26) peut être bloquée par rapport à la deuxième extrémité (13) à des intervalles définis (Bi = Σ bj ; j = 1 à M).

5. Dispositif selon la revendication 1, **caractérisé en ce que** la première pièce intermédiaire (22) comprend des moyens de positionnement (20), lesquels sont disposés sur une droite parallèle à l'axe central (19) et présentent successivement sur l'axe les distances cⱼ, de sorte que la face frontale inférieure (27) peut être bloquée par rapport à l'extrémité inférieure (15) à des intervalles définis (Ci = Σ cj ; j = 1 à Q).

6. Dispositif selon l'une quelconque des revendications 1 à 5, **caractérisé en ce que** les intervalles Aᵢ correspondent aux différentes épaisseurs normalisées d'un élément intermédiaire (2) du composant tibia d'une endoprothèse du genou.

7. Dispositif selon l'une quelconque des revendications 4 à 6, **caractérisé en ce que** les intervalles Bi correspondent aux différentes épaisseurs normalisées d'un espaceur (3) pour le composant fémur (1) d'une endoprothèse du genou.

8. Dispositif selon l'une quelconque des revendications 5 à 7, **caractérisé en ce que** les intervalles Ci peuvent être réglés conformément aux différentes dimensions A/P normalisées (30) d'un composant fémur (1) d'une endoprothèse du genou.

9. Dispositif selon l'une quelconque des revendications 1 à 8, **caractérisé en ce que**
A) la première pièce intermédiaire (22) comprend une extrémité supérieure (41) ainsi que, de manière contiguë sur l'axe entre la face frontale inférieure (27) et l'extrémité supérieure (41), une douille (40) et un tourillon (35),la douille (40) étant contiguë à la face frontale inférieure (27) et l'alésage de douille (43) étant ouvert vers la face frontale inférieure (27) ;
B) la deuxième pièce intermédiaire (21) comprend une extrémité arrière (42) ainsi que, de manière contiguë sur l'axe entre la face frontale avant (26) et l'extrémité arrière (42), une douille (40) et un tourillon (35),la douille (40) étant contiguë à la face frontale avant (26) et l'alésage de douille (43) étant ouvert vers la face frontale avant (26) ;
C) la première plaque (9) comprend, coaxialement à l'axe longitudinal de plaque (8), un premier alésage de guidage (44) pour recevoir la deuxième pièce intermédiaire (21) de manière coulissante axialement ;
D) la deuxième plaque (10) comprend, parallèlement à l'axe central (19), un deuxième alésage de guidage (45) pour recevoir la première pièce intermédiaire (22) de manière coulissante axialement ; et
E) les premier et deuxième palpeurs (16:11) comprennent chacun une broche-guide (46) respectivement située face à la première et à la deuxième surface d'appui (25 ; 24),
F) les broches-guides (46) pouvant être reçues dans les alésages de douille (43) en coulissement axial.

10. Dispositif selon la revendication 9, **caractérisé en ce que**
A) des rainures (36) ont été réalisées dans les tourillons (35) ainsi que dans les broches-guides (46), lesquelles forment les moyens de positionnement (20) et s'étendent respectivement transversalement à l'axe longitudinal de plaque (8) et à l'axe central (19) ;
B) le blocage axial des première et deuxième pièces intermédiaires (22 ; 21) par rapport aux première et deuxième plaques (9 ; 10) est assuré par des goupilles d'arrêt (31), lesquelles peuvent être reçues en coulissement axial dans des premiers alésages transversaux (32) ayant des axes d'alésage (34) s'étendant respectivement transversalement à l'axe longitudinal de plaque (8) et à l'axe central (19) dans les première et deuxième plaques (9 ; 10) et peuvent s'engager dans les rainures (36) sur les tourillons (35) ;
C) le blocage axial des premier et deuxième palpeurs (16 ; 11) par rapport aux première et deuxième pièces intermédiaires (22 ; 21) est assuré par des goupilles d'arrêt (31), lesquelles peuvent être reçues en coulissement axial dans des deuxièmes alésages transversaux (51) dans les première et deuxième pièces intermédiaires (22 ; 21) et peuvent s'engager dans les rainures (36) sur les broches-guides (46) ;
D) des trous oblongs (33), ayant des faces longitudinales respectivement parallèles à l'axe longitudinal de plaque (8) et à l'axe central (19), sont prévus dans les première et deuxième plaques (9; 10) pour la réception des goupilles d'arrêt (31), de sorte que les première et deuxième pièces intermédiaires (22:21) avec les premier et deuxième palpeurs (16; 11) bloqués peuvent néanmoins être déplacées par rapport aux première et deuxième plaques (9 ; 10) ; et
E) les goupilles d'arrêt (31) comprennent des évidements radiaux (38) avec des ergots (37) faisant saillie dans ces évidements (38),les ergots (37) n'occupant qu'une partie de la longueur axiale des évidements (38), de sorte que les goupilles d'arrêt (31) peuvent être déplacées dans une première position dans laquelle les ergots (37) sont engagés dans les rainures (36), et dans une deuxième position dans laquelle les ergots (37) sortent axialement des rainures (36) sous l'effet d'un déplacement axial des goupilles d'arrêt (31), et les tourillons (35) ou les broches-guides (46) peuvent être déplacés coaxialement à l'axe longitudinal de plaque (8), respectivement parallèlement à l'axe central (19).
